# EUROPEAN PATENT APPLICATION

(11) **EP 2 382 973 A1**
(43) Date of publication of application: **02.11.2011**
(21) Application number: 11003423.8
(22) Date of filing: 26.04.2011
(51) Int. Cl.: A61K 31/355, A61K 31/728, A61K 36/23, A61K 36/28, A61K 36/61, A61K 36/324, A61K 36/42, A61K 9/00, A61P 15/00

(54) **Composition of rectal suppositories based on natural extracts for the soothing and emollient treatment of the anorectal canal.**

(30) Priority: 29.04.2010 IT BO20100264
(71) Applicant: VI.RUS SRL, 70121 Bari (IT)
(72) Inventor: Russo, Vincenzo, 40126 Bologna (IT)

(57) **Abstract**

THE PRESENT INVENTION CONCERNS A PREPARATION FOR TOPICAL RECTAL USE AND ITS COMPOSITION.

THE PREPARATION OF THE PRESENT INVENTION CONTAINS PUMPKIN SEED OIL EXTRACT IN ASSOCIATION WITH OTHER NATURAL SUBSTANCES SUCH AS FOR EXAMPLE, BUT NOT ONLY, CENTELLA, HELICHRYSUM AND BOSWELLIA.

IN PARTICULAR, THE PREPARATION OF THE PRESENT INVENTION CAN BE USED IN THE SOOTHING AND EMOLLIENT TREATMENT OF THE ANORECTAL CANAL IN THE PRESENCE OF CONGESTIVE CONDITIONS ALSO DUE TO ACUTE AND CHRONIC PROSTATITIS AND BENIGN PROSTATIC HYPERTHROPHY FOR EXAMPLE, BUT NOT ONLY, IN THE FORM OF A SUPPOSITORY.

## Description

### FIELD OF THE INVENTION

The present invention concerns a preparation for topical rectal use and its composition. In particular, the preparation of the present invention can be used in the soothing and emollient treatment of the anorectal canal in the presence of congestive conditions also due to acute and chronic prostatitis and benign prostatic hypertrophy for example, but not only, in the form of a suppository.

### STATE OF THE ART

The main treatments currently available for the prostatic pathologies are oral drugs and food supplements and they are listed below:
ANTIBIOTICS: Antibiotic therapy is indicated for acute bacterial prostatitis and chronic bacterial prostatitis. In the acute forms, a therapy including broad-spectrum penicillins, as well as third generation cephalosporins and aminoglycosides for 10-14 days until disappearance of fever and other systemic symptoms is sufficient. In the chronic forms, longer cycles of 4-8 weeks are necessary. Depending on the sensitivity of the infecting microorganism, the combination therapy consists of a broad-spectrum fluoroquinolone (usually ciprofloxacin) as basic antibacterial, which is associated with a macrolide (usually azithromycin).

ANTI-INFLAMMATORIES: The use of anti-inflammatory drugs is indicated in case of chronic prostatitis; since the therapy consists of a prolonged period of administration (4-6 weeks, with the possibility of repeating a second cycle of treatment) it is preferred to use drugs that, if administered for long periods, do not cause any side effects in the stomach. It is preferred the use of cortisone anti-inflammatories (corticosteroids and glucocorticosteroids.

ALPHA-LYTICS: Alpha-adrenergic receptor blockers drugs also called alpha-blockers or alpha-lythics such as alfuzosin, tamsulosin, terazosin are administered in combination with antibiotic therapy in both acute and chronic prostatitis. Alpha-lythic therapy is based on the assumption that prostatitis and prostatic congestion are associated with and resulting from the difficulty in the passage of urine and semen through the urethra and in particular through the prostatic urethra due to the hypertonicity of its smooth muscles. Alpha-lytics act by relaxing the muscles of the bladder neck, prostatic urethra and of the same prostate, thus facilitating the passage of urine into the urethra.

ANTI-PROSTATICS: Anti-prostatics (such as finasteride and dutasteride) inhibit the activity of 5-alpha reductase, the enzyme that converts testosterone to its active metabolite, the dihydrotestosterone, which stimulates the growth of the prostate. Reducing the levels of dihydrotestosterone reduces the prostate enlargement.

One purpose of the present invention is to make a preparation which contains natural and not synthetic substances having the therapeutic effects as described above in a single product for topical rectal use for the prostatic pathologies.

### SUMMARY OF THE INVENTION

The present invention is set forth and characterized in the independent claims. The dependent claims describe other characteristics of the present invention or variants to the main inventive idea.

In accordance with the above purpose, a preparation for rectal use according to the present invention comprises the pumpkin seed oil extract (1).

It is known from scientific bibliography that the oils extracted from pumpkin seeds selectively inhibit the 5-alpha reductase thus blocking the conversion of testosterone into dihydrotestosterone (2). Because of its antiandrogen action, the inhibition of DHT is the main aim in the control of benign prostatic hypertrophy. In addition, the *β*-sitosterol, which is present in the oils extracted from pumpkin seeds, is a potent inhibitor of the biosynthesis of prostaglandin (PGE2), a mediator of inflammation that causes hyperemia and vascular congestion (3).

In accordance with some forms of embodiment of the present invention, the pumpkin seed oil extract is incorporated in a lipophilic excipient. The lipophilic excipient is advantageous in determining the structure of the preparation of the present invention, for example in the form of suppositories.

In some forms of embodiment, the pumpkin seed oil extract can be combined, in the preparation according to the present invention, with one or more other active substances selected from a group containing Centella, Helichrysum, Boswellia, Tea Tree oil, Vitamin E acetate and hyaluronic acid. As a whole the preparation has a beneficial and salutary effect on the rectal mucosa. Centella has an antiedemigene action (4), Helichrysum and Boswellia (5) give the preparation anti-inflammatory properties and the Teat Tree oil has a mild antibacterial action (6). In addition, hyaluronic acid and Vitamin E acetate have soothing and emollient properties (7, 8).

In some forms of embodiment, the active substances of the present invention, in particular the pumpkin seed oil extract, are incorporated into a suppository provided for the purpose and made to a large extent based on said lipophilic excipient.

The chosen lipophilic excipient has the property that it melts quickly so that it can be easily eliminated from the body. A lipophilic excipient which is commonly used is semi-synthetic glycerides which have these advantageous properties. Semi-synthetic glycerides are a mass of triglycerides of hydrogenated fatty acids deriving from palm oil and palm heart oil, with the shape and color of a waxy white pellet and a melting point of between about 34°C and 36°C. When the lipophilic excipient melts and is eliminated, the active substances, in particular the pumpkin seed oil extract, stay in situ to interact with the mucosa.

In some forms of embodiment, for the production of the preparation according to the present invention a quantity of pumpkin seed oil extract is used with a percentage in weight comprised between about 1.5% and about 12% with respect to the overall preparation.

In forms of embodiment of a suppository with a weight comprised between about 2 g and about 5 g, a quantity of pumpkin seed oil extract comprised between about 30 mg and about 240 mg is used.

In some forms of embodiment, for the production of the preparation according to the present invention a quantity of Centella is used with a percentage in weight comprised between about 1% and about 5% with respect to the overall preparation.

In forms of embodiment of a suppository with a weight comprised between about 2 g and about 5 g, a quantity of Centella comprised between about 20 mg and about 100 mg is used.

In some forms of embodiment, for the production of the preparation according to the present invention a quantity of Helichrysum is used with a percentage in weight comprised between about 1% and about 5% with respect to the overall preparation.

In forms of embodiment of a suppository with a weight comprised between about 2 g and about 5 g, a quantity of Helichrysum comprised between about 20 mg and about 100 mg is used.

In some forms of embodiment, for the production of the preparation according to the present invention a quantity of Boswellia is used with a percentage in weight comprised between about 1% and about 5% with respect to the overall preparation.

In forms of embodiment of a suppository with a weight comprised between about 2 g and about 5 g, a quantity of Boswellia comprised between about 20 mg and about 100 mg is used.

In some forms of embodiment, for the production of the preparation according to the present invention a quantity of Teat Tree oil is used with a percentage in weight comprised between about 0.05% and about 0.3% with respect to the overall preparation.

In forms of embodiment of a suppository with a weight comprised between about 2 g and about 5 g, a quantity of Teat Tree oil comprised between about 1 mg and about 6 mg is used.

In some forms of embodiment, for the production of the preparation according to the present invention a quantity of Vitamin E acetate is used with a percentage in weight comprised between about 0.05% and about 0.4% with respect to the overall preparation.

In forms of embodiment of a suppository with a weight comprised between about 2 g and about 5 g, a quantity of Vitamin E acetate comprised between about 1 mg and about 8 mg is used.

In some forms of embodiment, for the production of the preparation according to the present invention a quantity of Hyaluronic acid is used with a percentage in weight comprised between about 0.05% and about 0.3% with respect to the overall preparation.

In forms of embodiment of a suppository with a weight comprised between about 2 g and about 5 g, a quantity of Hyaluronic acid comprised between about 1 mg and about 6 mg is used.

The form of embodiment and application by means of suppositories is particularly advantageous because it allows the active substances, in particular the pumpkin seed oil extract, to be kept in the anorectal canal for a long period and released gradually.

The above mentioned lipophilic excipients are useful because they give to the suppository, the right consistency and influence positively the release of the active substances, in particular the pumpkin seed oil extract.

A preparation as described above also comes within the scope of the present invention, for use in the soothing and emollient treatment of the anorectal canal in the presence of congestive conditions also due to acute and chronic prostatitis and benign prostatic hypertrophy for example, but not only, in the form of a suppository.

### DESCRIPTION OF SOME PREFERENTIAL FORMS OF EMBODIMENT

For suppositories with a weight between about 2 g and about 5 g, formed for the most part by a lipophilic excipient, for example semi-synthetic glycerides, significant results can be achieved in the treatment of anorectal mucosa, using the following quantities of active substances added to the lipophilic excipient:
- pumpkin seed oil extract: from about 30 mg to about 240 mg;
- Centella: from about 20 mg to about 100 mg;
- Helichrysum: from about 20 mg to about 100 mg;
- Boswellia: from about 20 mg to about 100 mg;
- Tea Tree oil: from about 1 mg to about 6 mg;
- Vitamin E acetate: from about 1 mg to about 8 mg;
- hyaluronic acid: from about 1 mg to about 6 mg.

One form of embodiment particularly preferred in the present invention provides to make a suppository which weighs about 2 g and which uses, as active ingredients, a mixture which contains the following quantities of substances:
- pumpkin seed oil extract: about 120 mg;
- Centella: about 40 mg;
- Helichrysum: about 40 mg;
- Boswellia: about 40 mg;
- Tea Tree oil: about 2 mg;
- Vitamin E acetate:about 4 mg;
- hyaluronic acid: about 2 mg.

In this form of embodiment, to obtain a suppository of about 2 g, a quantity of about 1752 mg of semi-synthetic glycerides is used as lipophilic excipient.

### BIBLIOGRAPHY

1. Cucurbita Pepo. L. Bombardelli E., Morazzoni P. Fitoterapia Vo. LXVIII, No 4, 1997
2. Inhibition of testosterone-induced hyperplasia of the prostate of sprague-dawley rats by pumpkin seed oil. Gossell-Williams M, Davis A, O'Connor N. J Med Food. 2006 Summer; 9(2):284-6
3. Treatment of benign prostatic hyperplasia with phytosterols. Carbin BE, Larsson B, Lindahl O. Br J Urol. 1990 Dec; 66(6):639-41
4. Phytotherapeutics: an evaluation of the potential of 1000 plants. Cravotto G, Boffa L, Genzini L, Garella D. J Clin Pharm Ther. 2010 Feb;35(1):11-48
5. Boswellic acids -components of frankincense- as the active principle in treatment of chronic inflammatory diseases. Ammon HP. Wien Med Wochenschr. 2002; 152(15-16):373-8
6. Melaleuca alternifolia -Tea Tre- Oil: a Review of Antimicrobial and Other Medicinal Properties. C.F. Carson, K.A. Hammer, and T.V. Riley. Clinical Microbiology Reviews, Jan. 2006, p. 50-62 Vol. 19, No. 1
7. Topical vitamins. Burgess C. J Drugs Dermatol. 2008 Jul; 7(Suppl):s2-6
8. Beneficial actions of exogenous hyaluronic acid on wound healing. King SR. Hickerson WL, Proctor KG. Surgery 1991 Jan; 109(1):76-84

## Claims

1. Preparation for topical rectal use comprising pumpkin seed oil extract.

2. Preparation as in claim 1, **characterized in that** the pumpkin seed oil extract is incorporated in a lipophilic excipient.

3. Preparation as in claim 2, **characterized in that** said lipophilic excipient comprises semi-synthetic glycerides.

4. Preparation as in claim 2 or 3, **characterized in that** the preparation is in the form of a rectal suppository formed by said lipophilic excipient, suitable to be introduced into the rectal canal.

5. Preparation as in any one of the preceding claims, **characterized in that** for the production thereof a quantity of pumpkin seed oil extract is used with a percentage in weight with respect to the overall preparation comprised between about 1.5% and about 12%.

6. Preparation as in any one of the preceding claims, **characterized in that** it further comprises one or more other active substances selected from a group comprising Centella, Helichrysum, Boswellia, Tea Tree oil, Vitamin E acetate and hyaluronic acid.

7. Preparation as in claim 6, **characterized in that** for the production thereof a quantity of Centella is used with a percentage in weight with respect to the overall preparation comprised between about 1% and about 5%.

8. Preparation as in claims 6 or 7, **characterized in that** for the production thereof a quantity of Helichrysum is used with a percentage in weight with respect to the overall preparation comprised between about 1% and about 5%.

9. Preparation as in claims 6, 7 or 8, **characterized in that** for the production thereof a quantity of Boswellia is used with a percentage in weight with respect to the overall preparation comprised between about 1% and about 5%.

10. Preparation as in claims 6, 7, 8 or 9, **characterized in that** for the production thereof a quantity of Tea Tree oil is used with a percentage in weight with respect to the overall preparation comprised between about 0.05% and about 0.3%.

11. Preparation as in claims 6, 7, 8, 9 or 10, **characterized in that** for the production thereof a quantity of Vitamin E acetate is used with a percentage in weight with respect to the overall preparation comprised between about 0.05% and about 0.4%.

12. Preparation as in any one of claims from 6 to 11, **characterized in that** for the production thereof a quantity Hyaluronic acid is used with a percentage in weight with respect to the overall preparation comprised between about 0.05% and about 0.3%.

13. Preparation as in any one of the preceding claims for use in the soothing and emollient treatment of the anorectal canal in the presence of congestive conditions also due to acute and chronic prostatitis and benign prostatic hypertrophy.
